# EUROPEAN PATENT APPLICATION

(11) **EP 2 738 164 A1**
(43) Date of publication of application: **04.06.2014**
(21) Application number: 13194302.9
(22) Date of filing: 25.11.2013
(51) Int. Cl.: C07D 301/14, C07D 303/14, C07D 303/16, C07C 237/04

(54) **Synthesis of an intermediate of an antiviral compound**

(30) Priority: 29.11.2012 IT MI20122036
(71) Applicant: Dipharma Francis S.r.l., 20021 Baranzate (MI) (IT)
(72) Inventor: Allegrini, Pietro, 20021 Baranzate (MI) (IT); Brunoldi, Enrico, 20021 Baranzate (MI) (IT); Attolino, Emanuele, 20021 Baranzate (MI) (IT); Bove, Alessio, 20021 Baranzate (MI) (IT)
(74) Representative: Bertuccio, Silvia

(57) **Abstract**

Process for the preparation of a cyclopropylamide compound (II) which is useful as a structural unit in a process for the preparation of a viral protease inhibitor.

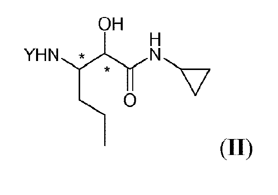

## Description

The present invention relates to a process for the preparation of an intermediate useful in the preparation of a viral protease inhibitor.

### PRIOR ART

(1S, 3aR, 6aS)-2-[(2S)-2-[[(2S)-2-cyclohexyl-2-[(2-pyrazinylcarbonyl) amino]acetyl]amino-3,3-dimethylbutanoyl]-N-[(1S)-1-[(cyclopropylamino) (oxo)acetyl]butyl]-3,3a,4,5,6,6a-hexahydro-1H-cyclopenta[c]pyrrole-3-carboxy amide of formula **(I),** also known as telaprevir, is a potent viral protease inhibitor used to treat hepatitis C infections.

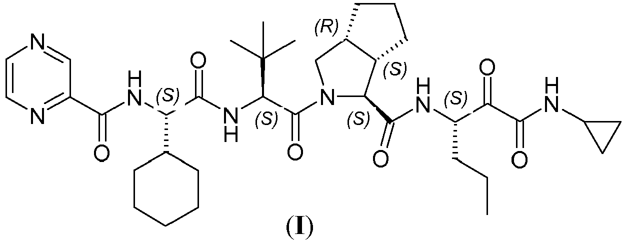

The preparation of telaprevir is reported in US 7,820,671, and involves the assembly of 6 different structural units, with the creation of five amide bonds as reported below in **Scheme 1** 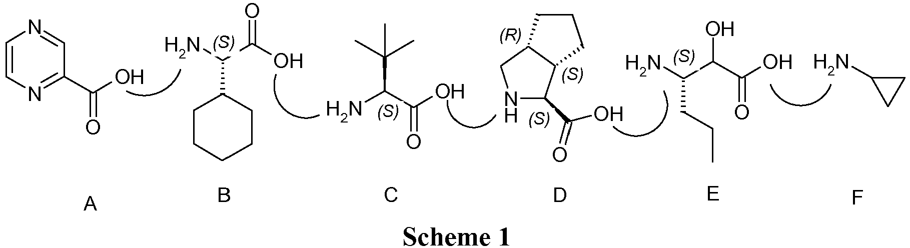 and subsequent oxidation of the alcoholic hydroxyl of the structural unit of formula E. The structural units of formula A and F are pyrazine-carboxylic acid and cyclopropylamine respectively.

The structural units of formula B, C, D and E are amino acids, with the (S) configuration.

The compounds of formula B and C are the commercially available amino acids (S)-cyclohexylglycine and (S)-tert-leucine respectively. The compounds of formula D and E are synthetic amino acids with a more complex structure.

The preparation of the key amino acid of formula D, although rather complex, has been effected by various methodologies, such as those described in US 7,820,671 and US 7,776,887.

The preparation of the compound of formula EF is reported, for example, in US 7,776,887, starting from L-norvaline, according to two different synthesis sequences. The first, **Scheme 2,** known as the cyanohydrin method, involves protecting the amino function of L-norvaline as benzyloxycarbonyl (Cbz) to give (E1), which is then converted to the corresponding Weinreb amide (E2) by treatment with methoxymethylamine in the presence of a condensing agent. The amide (E2) is then treated with lithium aluminium hydride or diisobutyl aluminium monohydride to give protected norvalinal (E3). By reacting (E3) with an alkaline cyanide or acetone cyanohydrin, cyanohydrin (E4) is obtained which, after hydrolysis and reprotection, provides the acid (E5). The final cyclopropylamide (EF), ready to be condensed with amino acid (D), is obtained once again by condensation between acid (E5) and cyclopropylamine (F) in the presence of a condensing agent and subsequent deprotection of the amino function, Scheme 2.

Even if the nasty cyclopropylamine, a bad-smelling low boiling amine, is used in the very last stage of the synthesis, unfortunately the condensation reaction is carried out with a complex, expensive condensing agent, and produces very modest yields of the desired product. The preparation of the aldehyde (E3) is also rather laborious, and requires the use of the expensive methoxymethylamine.

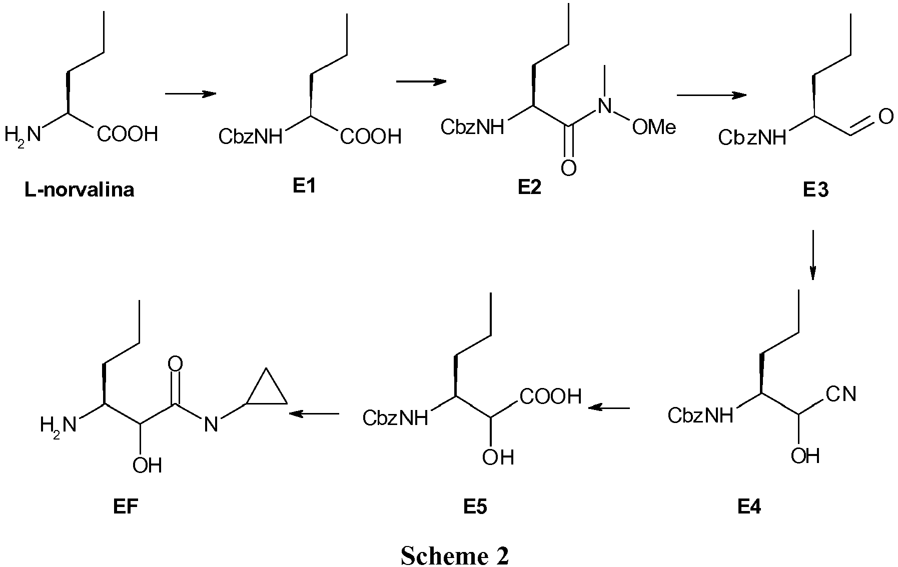

The second synthesis strategy, **Scheme 3,** attempts to circumvent the obstacle of using toxic cyanide, bad-smelling cyclopropylamine and the low yield of the condensation between the acid (E5) and cyclopropylamine (F), by reacting the key aldehyde (E3) with cyclopropyl isocyanide in the presence of trifluoroacetic acid, and subsequent deprotection, Scheme 3. In this way amide (EF) is obtained with a good yield, but the use of a low-molecular-weight isocyanide like cyclopropyl isocyanide involves major safety problems which make its use on an industrial scale impractical.

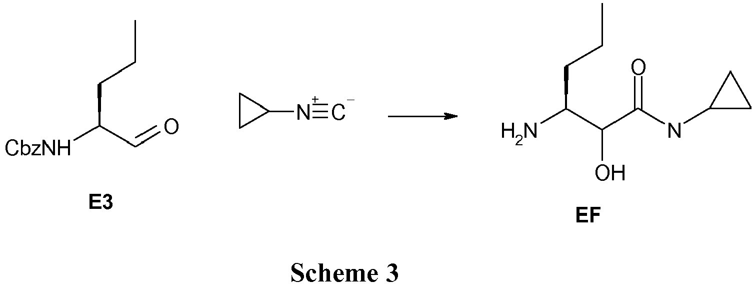

Some other processes directed to the preparation of the intermediate EF, making use of cyclopropylamine at the beginning of the synthetic sequence have been reported in literature, but it has to be noted that using cyclopropylamine at the beginning of the synthetic sequence, which means using larger amounts of amine, makes the process harmful for workers, hazardous for the environment and more expensive from an industrial point of view also because of extensive amounts of wastes which are to be disposed.

There is consequently a need for a more advantageous alternative method of preparing structural unit (EF), so as to improve the telaprevir preparation process. Said novel method should solve in particular all the above mentioned chemical and technical problems in order to be more industrially scalable, involve the use of cheaper, easier to handle reagents and mild reaction conditions, and at the same time provide high yields of the desired compounds.

### SUMMARY OF THE INVENTION

A process that eliminates said problem has been found. The invention provides a process for the preparation of a compound of formula **(II),** as a single stereoisomer or a mixture of stereoisomers or a salt thereof, 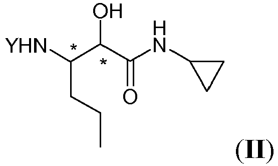 comprising the reaction of a compound of formula (III) or a salt thereof 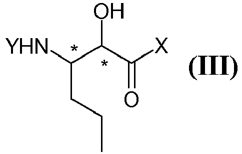 with the cyclopropylamine of formula (IV), or a salt thereof, 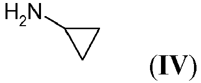 wherein an intermediate of formula (V) 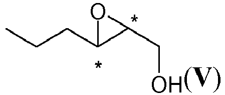 is obtained by epoxidation reaction of a compound of formula (VI) 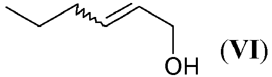 wherein the double bond has the Z or E configuration.

The same process, in a preferred aspect thereof, also comprises the use of a compound of formula (V) in optically active form, 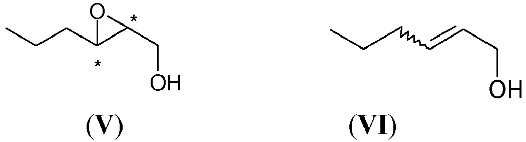 wherein the stereocentre, indicated by asterisk * in the 3 position, has the (S) or (R) absolute configuration, as intermediate in the preparation of a compound of formula **(III),** obtained by a process comprising enantioselective epoxidation of a compound of formula **(VI),** wherein the double bond has the Z or E configuration.

### DETAILED DESCRIPTION OF THE INVENTION

The subject of the present invention is a process for the preparation of a compound of formula **(II)** as a single stereoisomer or mixture of stereoisomers, or a salt thereof, 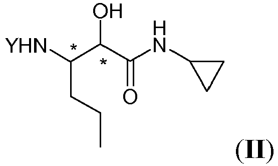 wherein Y is H or an amino protecting group and asterisk * indicates the presence of a stereocentre with the (R) or (S) configuration or a racemic mixture thereof; comprising the reaction of a compound of formula **(III)** or a salt thereof 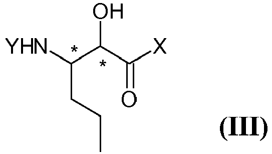 wherein X is an -OR₁ group wherein R₁ is an optionally substituted C₁-C₆ alkyl group, an optionally substituted aryl group or an optionally substituted heteroaryl group; or X is an -SR₂ group wherein R₂ is an optionally substituted C₁-C₆ alkyl group, an optionally substituted aryl group or an optionally substituted heteroaryl group; or X is the reactive residue of a carboxylic acid; and Y and asterisk * are as defined above; with the cyclopropylamine of formula **(IV),** or a salt thereof, 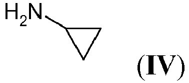 and, if applicable, the conversion of a compound of formula **(II)** to another compound of formula **(II)** or a salt thereof; which said process comprises the use of a compound of formula **(V)** as starting material, 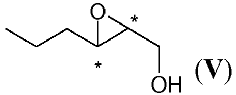 wherein asterisk * is as defined above, obtained by epoxidation of a compound of formula (VI), 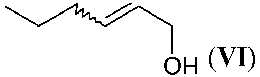 wherein the double bond has the Z or E configuration.

It has been surprisingly found that the process of the present invention, making use of the intermediate of formula **(V)** in the preparation of the intermediate of formula **(II),** is highly advantageous since the process to obtain the desired compound of formula **(II)** have been found to be safe and efficient.

In particular, according to the reaction scheme of the present invention, a compound of formula **(V)** can be converted to a compound of formula **(VIII),** which is then converted to a compound of formula **(III)** using mild reaction conditions. The reaction scheme of the present invention is particularly advantageous since, contrary to what expected, the instant sequence of the steps and the reactants used render the process safe and cheaper from an industrial point of view. Such process for the preparation of the intermediate of formula **(II)** and therefore of Telaprevir of formula **(I)** is thus unexpectedly advantageous.

The stereocentres, indicated by asterisk *, in the compounds of formula **(II)** and **(III),** can be (R) or (S) or a racemic mixture thereof.

A salt of a compound of formula **(II)** or **(III)** is, for example, a pharmaceutically acceptable salt.

A C₁-C₆ alkyl group, which may be straight or branched, is preferably a C₁-C₄ alkyl group, preferably methyl, ethyl, isopropyl or tert-butyl, optionally substituted by one or more substituents, typically one to three, selected independently from phenyl and halogen, such as fluorine, chlorine and iodine, preferably fluorine. Said group is more preferably a C₁-C₄ alkyl group, in particular methyl or ethyl, optionally substituted by phenyl.

An aryl group, which can be partly saturated, is, for example, phenyl or naphthyl, preferably phenyl, optionally substituted by one or more substituents, preferably one to three, typically selected from C₁-C₆ alkyl optionally substituted by one to three halogen atoms, preferably fluorine, chlorine and bromine; C₁-C₆ alkoxy; nitro; cyano; halogen, preferably fluorine, chlorine or bromine.

A partly saturated heteroaryl group, which can be monocyclic or bicyclic, can contain one to four heteroatoms selected independently from nitrogen, oxygen and sulphur, and can be optionally substituted by one or more substituents, preferably one to three, preferably selected from fluorine, chlorine and bromine; C₁-C₆ alkoxy; nitro; cyano; halogen, preferably fluorine, chlorine or bromine.

Examples of heteroaryl groups are pyrrole, pyridine, benzofuran, thionaphthene, indole, quinoline, isoquinoline, pyrazole, imidazole, oxazole, isoxazole, thiazole, indazole, benzoimidazole, benzoxazole, benzothiazole, pyridazine, pyrimidine, quinazoline, pyrazine, 1,2,3-triazole, 1,2,4-triazole, 1,2,3,4-tetrazole and 1,2,3-benzotriazole, bonded to the oxygen or sulphur atom of the -OR₁ or -SR₂ group via a bond with one of their carbon atoms or via a bond with one of their heteroatoms.

A group Y as amino protecting group can, for example, be one of the protecting groups of said function known from peptide chemistry, such as the tert-butyl carbamate (Boc), 9-fluorenylmethyl carbamate, benzyl carbamate (Cbz), acetamide, trifluoroacetamide or p-toluene sulphonamide groups. The amino function can be protected and deprotected at any stage of the process, depending on which is most convenient for the process. The protection is preferably effected immediately before the reaction, and deprotection immediately after the reaction.

The reactive residue X of a compound of formula **(III)** is known to be a good leaving group. Examples of reactive residues X are, in particular, halogen, preferably chlorine; imidazole; or an -OCORa or -OCOORa group, wherein Ra is a straight or branched C₁-C₆ alkyl group, preferably C₁-C₄ alkyl, optionally substituted, for example, by phenyl or halogen, such as chlorine or fluorine.

The reaction between a compound **(III)** and a compound **(IV)** proceeds with complete retention of the absolute configuration of the stereocentres indicated by asterisk *. The absolute configuration of the stereocentre of a compound **(II)** or a salt thereof will therefore be the same as compound **(III)** used in the reaction.

If applicable, the reaction can be conducted in the presence of a solvent which can be, for example, a polar aprotic solvent, typically an amide, such as dimethylformamide, dimethylacetamide or N-methylpyrrolidone, preferably dimethylacetamide, acetonitrile or dimethyl sulphoxide; or an acyclic or cyclic ether, such as methyl tert-butyl ether, tetrahydrofuran or dioxane; a chlorinated solvent, such as dichloromethane, dicloroethane, chloroform or chlorobenzene; an apolar aprotic solvent, typically toluene; a polar protic solvent, typically a straight or branched C₁-C₈ alkanol, such as a C₁-C₅ alkanol like methanol, ethanol, isopropanol, tert-butanol; water; a tertiary amine, such as triethylamine or a mixture of two or more, preferably two or three, of said solvents.

In a preferred embodiment, the reaction between a compound of formula **(III)** and a compound of formula **(IV)** is carried out in the presence of methanol or tetrahydrofuran.

The reaction can be conducted at a temperature of between about 0°C and the solvent reflux temperature, preferably between about 40°C and about 80°C.

The reaction can be conducted at atmospheric pressure or preferably in a closed reactor so that the reaction takes place under pressure, for example between about 1.5 and 5 bar.

A compound of formula **(II)** can be converted to another compound of formula **(II)** according to known methods.

For example, a compound of formula **(II),** wherein Y is an amino protecting group, can be converted to a compound of formula **(II)** wherein Y is H, according to methods known to the prior art for the deprotection of said function.

Similarly, a compound of formula **(II),** wherein Y is H, can be converted to a compound of formula **(II),** wherein Y is an amino protecting group, according to known methods. Similarly, a compound of formula **(II)** can be converted to a salt thereof, or vice versa, according to known methods.

A compound of formula **(II),** as defined above, wherein the stereocentre, indicated by asterisk * in the 3 position, has the (S) absolute configuration, can be usefully employed in a process for the preparation of telaprevir of formula (I), for example by the synthesis method reported in US 7,820,671.

A further subject of the invention is therefore a process for the preparation of a compound of formula **(I)** or a pharmaceutically acceptable salt thereof, comprising the use as starting material of a compound of formula **(II),** wherein Y is as defined above, and wherein the stereocentre, indicated by asterisk * in the 3 position, has the (S) absolute configuration, or a salt thereof, obtained by the process according to the present invention.

A compound of formula (III) as defined above, and similarly a compound of formula (**IIIa**) 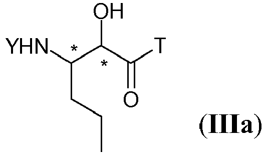 wherein Y and asterisk * are as defined above, and T is OH, -OR₁ or -SR₂, wherein R₁ and R₂ are as defined above, can be obtained by a process comprising reduction of the azido group in a compound of formula **(VII)** 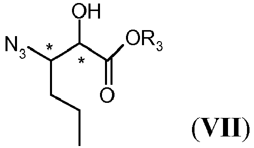 wherein R₃ is H or a C₁-C₆ alkyl group and asterisk * is as defined above, and if applicable the conversion of a compound of formula (**IIIa**)thus obtained to another compound of formula (**IIIa**)or formula **(III).**

The reduction of said azido group to a compound of formula **(VII)** can be conducted by known methods, for example by catalytic hydrogenation or treatment with PPh₃ and water.

A compound of formula (**IIIa**)can be converted to another compound of formula (**IIIa**)or **(III)** according to known methods.

For example, a compound of formula (**IIIa**), as defined above, wherein T is -OR₁, wherein R₁ and Y are as defined above, can be converted to another compound of formula (**IIIa**)wherein Y is as defined above and T is OH, or vice versa, according to known methods.

A compound of formula **(III),** wherein X is the reactive residue of a carboxylic acid, can be prepared from a compound of formula (**IIIa**), wherein T is OH, by reaction with an activating agent, according to known methods. Examples of activating agents are thionyl chloride; an acyl chloride, such as acetyl chloride or pivaloyl chloride; an alkyl chloroformate, such as ethyl or isopropyl chloroformate; or carbonyl diimidazole (CDI).

A compound of formula **(VII)** can be prepared by a process comprising the reaction of an epoxy compound of formula **(VIII)** 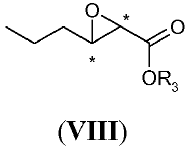 wherein R₃ and asterisk * are as defined above, with an alkali or alkaline-earth metal azide, preferably sodium, and optionally in the presence of a Lewis acid, in a solvent, and if applicable the conversion of a compound of formula **(VII)** to another compound of formula **(VII).**

An azide is preferably an azide of an alkali metal, more preferably sodium.

A Lewis acid is preferably a lithium or magnesium salt, such as lithium chlorate or magnesium sulphate.

A compound of formula **(VII),** wherein R₃ is a C₁-C₆ alkyl group, can be converted to a compound of formula **(VII)** wherein R₃ is H, or vice versa, according to known methods.

A compound of formula **(VIII)** wherein R₃ is H can be prepared by a process comprising oxidation of a compound of formula (V) 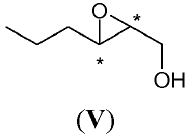 wherein asterisk * is as defined above.

Said oxidation reaction can be conducted by conversion directly from the hydroxyl to carboxyl group; or through the aldehyde intermediate of formula **(IX)**

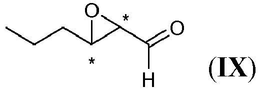

Direct oxidation can be conducted, for example, by treating a compound of formula **(V)** with a hexavalent chromium derivative, such as CrO₃, in sulphuric acid (Jones reagent), PDC (pyridinium dichromate) or PCC (pyridinium chlorochromate); or using ruthenium-based metal catalysts in the presence of an oxidising agent such as sodium periodate or periodic acid.

Alternatively, oxidation can be carried out via the intermediate aldehyde of formula **(IX).** The aldehyde can be isolated or not, depending on the experimental conditions used.

When a compound of formula **(V)** is converted to a compound of formula **(VIII)** via the aldehyde of formula **(IX),** it can also be prepared by methods known to the prior art, for example by the activated dimethyl sulphoxide (DMSO) technique. DMSO can be activated in the presence of an acyl chloride or an anhydride or the pyridine-sulphuric anhydride complex, according to the well-known Swern oxidation conditions, and subsequently the aldehyde of formula **(IX)** can be oxidised to the compound of formula **(VIII),** for example by using the above-mentioned direct oxidation conditions from alcohol to acid or by methods known to the prior art for the transformation of the aldehyde functional group to carboxyl, for example by treatment with silver nitrate or dilute aqueous nitric acid.

According to a preferred aspect of the invention, a compound of formula **(V)** can be converted to a compound of formula **(VIII)** by oxidation with an oxidising agent catalysed by TEMPO [(2,2,6,6-tetramethylpiperidin-1-yl)oxyl].

An oxidising agent can be, for example, a salt of formula MZOₙ, wherein M is an alkali or alkaline-earth metal and Z is a halogen atom, preferably chlorine or bromine, and n can be 1 or 2.

The oxidation reaction can be carried out by at least stoichiometric treatment with sodium chlorite and catalytic or stoichiometric quantities of sodium hypochlorite and TEMPO in catalytic quantities in a solvent mixture.

Sodium chlorite can be used, for example, in a molar ratio with the compound of formula **(V)** between 5 and 1, preferably between 2.5 and 1.

Sodium hypochlorite can be used in stoichiometric or catalytic quantities, for example in a molar ratio with the substrate ranging between 100% and 1%, preferably in catalytic quantities, for example in a molar ratio ranging between 20% and 1%.

The TEMPO used in the oxidation reaction can be employed in homogenous or heterogeneous phase, and can be free or supported on a solid phase, covalently bonded or only adsorbed, for example on silica.

TEMPO can be used in a molar ratio with the substrate ranging between 20% and 1%, preferably between 5% and 1%.

The reaction can be conducted at a temperature between the solvent reflux temperature and -5°C, preferably between 50°C and 0°C.

A solvent mixture is a mixture formed, for example, by a solution comprising an aqueous buffer with a pH between about 3.0 and 9.0, more preferably around a pH of about 7.0; and possibly an organic co-solvent, miscible or immiscible with the buffer.

A solution of an aqueous buffer may, for example, be a known phosphate buffer, ammonium bicarbonate, ethanolamine/HCl; the reaction is preferably conducted in phosphate buffer.

An organic co-solvent may, for example, be a polar aprotic solvent such as dimethylformamide, dimethylacetamide, acetonitrile or dimethyl sulphoxide; a ketone, such as acetone or methyl isobutyl ketone; an ether, such as tetrahydrofuran or dioxane; an apolar aprotic solvent such as toluene, or a halogen solvent such as dichloromethane, chloroform or dicloroethane, preferably a polar aprotic solvent.

A compound of formula **(V)** can be prepared by epoxidation reaction of a compound of formula **(VI),** 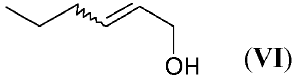 wherein the configuration of the olefin can be E or Z.

Said epoxidation reaction can be conducted, for example, by treatment with an oxidizing agent, typically a peroxyacid such as m-chlorine perbenzoic acid, or an organic peroxide such as tert-butyl hydroperoxide, cumyl hydroperoxide or trityl hydroperoxide, or hydrogen peroxide in the presence of acetic acid or of a catalyst consisting of a complex of a transition metal, such as sodium metavanadate or sodium tungstate.

A compound of formula **(V),** and therefore a compound of formula **(VIII),** prepared from a compound of formula **(VI)** by the methods reported above, are obtained in racemic form.

For this reason, when a compound of formula **(II)** or **(III),** as defined above, is obtained from a compound of formula **(VIII),** as described above, they are obtained in racemic form in terms of stereochemistry.

The compound of formula **(VII),** obtained as described above, presents an absolute configuration at the stereocentre in the 3 position, opposite to the one present in the precursor epoxides of formula **(V)** or **(VIII).**

Consequently, by using a compound of formula **(V)** or **(VIII)** in the appropriate optically active form, a compound of formula **(II),** as defined above, is obtained, wherein the stereocentre in the 3 position has the **(S)** absolute configuration, which is useful as an intermediate in the preparation of telaprevir.

The resolution of a compound of formula **(VIII)** to an optically active form thereof can be conducted by crystallisation via diastereomeric salts, obtained from a compound of formula **(VIII),** wherein R₃ is H, with a chiral amine.

It has also been found that a compound of formula **(VIII),** wherein R₃ is H, in optically active form, can be advantageously obtained by oxidation of an epoxide of formula **(V)** already in optically active form.

A compound of formula **(V),** already in optically active form, can be prepared by a process comprising the enantioselective epoxidation reaction of a compound of formula **(VI)**

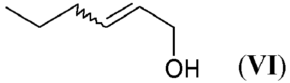

in the presence of an oxidising agent and of a complex of a titanium salt with an optically active ligand.

A titanium salt, such as titanium isopropylate, can be used in stoichiometric quantity or in catalytic quantity, preferably in a molar ratio with the substrate ranging between about 20% and 1%, more preferably ranging between about 10% and 1%.

An optically active ligand can be an optically active diol, such as a derivative of tartaric acid, preferably an ester thereof, e.g. methyl tartrate, diethyl tartrate or isopropyl tartrate.

An optically active ligand can be used in stoichiometric or catalytic quantity, preferably in a molar ratio with the substrate ranging between about 20% and 1%, more preferably ranging between about 10% and 1%.

The epoxidation reaction can be conducted at a temperature between about the reflux temperature of the solvent and about -40°C, preferably between about 0°C and about -40°C. For example, the reaction temperature can be gradually increased from about -20°C to about 0°C during the reaction.

The reaction can be conducted in a solvent inert under the reaction conditions, such as a halogenated solvent like dichloromethane, chloroform or dicloroethane; an apolar aprotic solvent, preferably an aromatic hydrocarbon such as benzene, toluene or xylene, or an aliphatic hydrocarbon such as hexane, heptane or iso-octane; a polar aprotic solvent such as dimethylformamide, dimethylacetamide, acetonitrile or dimethyl sulphoxide; a ketone, such as acetone or methyl isobutyl ketone; an ether, such as tetrahydrofuran or dioxane or a mixture of two or more, preferably two or three, of said solvents. The reaction is preferably conducted in dichloromethane or toluene.

The oxidising agent can be a peroxide such as tert-butyl hydroperoxide, cumyl hydroperoxide or trityl hydroperoxide.

The oxidising agent can be used in stoichiometric quantity or in excess, preferably in a molar ratio with the substrate ranging between about 5 and 1, more preferably ranging between about 2 and 1.

If applicable, the reaction can be conducted in the presence of desiccant agents, for example in the presence of molecular sieves with a porosity of 3Å or 4 Å, in powdered or granule form, or in the presence of anhydrous salts such as sodium sulphate or magnesium sulphate.

The absolute stereochemistry of the stereocentre, indicated by asterisk * in the 3 position of the compound of formula **(V)** thus obtained, can depend both on the absolute configuration of the chiral ligand used and on the geometry of the olefin used as reaction substrate.

In particular, if the reaction is conducted on an olefin of formula **(VI)** with the *E* configuration, an epoxide with the 3-(R) absolute configuration will mainly be obtained by using diethyl tartrate in the D configuration, or 3-(S) using diethyl tartrate in the L configuration. Conversely, if the reaction is conducted on an olefin of formula **(VI)** with the Z configuration, an epoxide with the 3-(R) absolute configuration will mainly be obtained by using a tartrate with the L configuration, or 3-(S) using a tartrate with the D configuration.

By conducting the reaction under the conditions described above, the compound of formula **(V)** can be obtained with an enantiomeric excess exceeding 90%.

This result is far more advantageous than methods comprising the resolution of a compound of formula **(VIII)** to an optically active form thereof. In fact, these methods generally produce disappointing results, as the products obtained have such low enantiomeric excesses that they cannot be used in the continuance of the industrial synthesis. Moreover, the classic method of crystallisation via diastereomeric salts intrinsically produces reaction yields below 50%, which means that the process involves high costs and the need to use substantial quantities of starting materials.

A compound of formula **(V),** wherein the stereocentre in the 3 position as defined above has the (R) or (S) configuration, can be oxidised to give a compound of formula **(VIII)** with complete retention of the absolute stereochemistry of the stereocentre.

An enantiomer of a compound of formula **(V),** wherein the stereocentre in the 3 position has the (R) configuration, obtained as described above, can be advantageously used in a process for the preparation of telaprevir of formula **(I).**

Said compound of formula **(V),** in the form of an enantiomer thereof, wherein the stereocentre in the 3 position has the (R) configuration, can be converted to an optically active compound of formula **(VIII),** which in turn is converted to an optically active compound of formula **(VII),** and finally to a compound of formula **(III),** wherein Y is hydrogen and X is an -OR₁ group wherein R₁ is as defined above, wherein the stereocentre in the 3 position has the absolute (S) configuration), which is useful as an intermediate in the preparation of telaprevir.

Thus according to a preferred aspect thereof, the invention provides a method for the preparation of a compound of formula **(II),** as defined above, comprising the reaction of a compound of formula **(III)** with a compound of formula **(IV),** as defined above, wherein a compound of formula **(V)** is obtained by a process comprising enantioselective epoxidation of a compound of formula **(VI)** 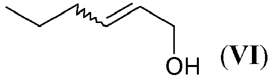 by reaction with an organic peroxide, in the presence of a titanium salt and an optically active ligand.

Preferably, a compound of formula (III) is obtained according to said method wherein Y is hydrogen and X is an -OR₁ group, wherein R₁ is as defined above, wherein the stereocentre in the 3 position has the (S) absolute configuration, which is useful as an intermediate in the preparation of telaprevir.

A further purpose of the present invention is therefore a process for the preparation of a compound of formula **(I),** or a pharmaceutically acceptable salt thereof, comprising the use as starting material of a compound of formula **(V),** wherein the stereocentre in the 3 position has the (R) configuration, obtained by enantioselective epoxidation of a compound of formula **(VI),** as described herein.

The following examples illustrate the invention.

### Example 1 - Synthesis of (3R-trans)-(3-propyloxyranyl)methanol (V)

MS 4 Å (7.5 g, D-(-)-diethyltartrate (3.1 g, 15 mmol) and cumene hydroperoxide (80% w/w, 95 g, 500 mmol) are added in sequence to a solution of Ti(OiPr)₄ (3.55 g, 12.5 mmol) in dichloromethane (375 ml) cooled to -20°C under inert atmosphere, and the temperature is maintained below -15°C. After 30 minutes a solution of *trans*-2-hexen-1-ol (25 g, 250 mmol) in dichloromethane (125 ml) is added in 20 minutes, and the temperature is maintained below -15°C. After 90 minutes the reaction mixture is heated to 0°C and water (70 ml) is added, while maintaining the mixture under vigorous stirring. An NaOH solution (30%, 15 ml) is added to the mixture after 45 minutes, and the mixture is stirred vigorously for 30 minutes, after which the phases are separated. The aqueous phase is extracted twice with 50 ml of dichloromethane, and the combined organic phases are treated with a saturated solution of sodium thiosulphate (200 ml) for 30 minutes. The phases are separated and the organic phase is treated with a saturated solution of sodium chloride, dried on sodium sulphate, filtered and concentrated at low pressure until crude (3R-*trans*)-(3-propyloxyranyl)methanol of formula **(V)** is obtained, which is not purified but used "as is" in the next reaction.

A portion of the crude product is purified by flash chromatography for analysis purposes. The chemically pure sample thus obtained was analysed by NMR spectroscopy, and perfectly agreed with the structure. The same sample was also derivatised as Mosher ester and analysed by gas chromatography; it proved chemically pure, with an enantiomeric excess of 94%.

¹H-NMR, (CDCl₃) δ: 3.92 (dd, 1 H, *J* = 2.7, 12.8 Hz), 3.63 (dd, 1 H, *J* = 4.5, 12.8 Hz), 2.9-3.0 (m, 2 H), 1.4-1.6 (m, 4 H), 0.94 (t, 3 H, *J* = 8 Hz).

### Example 2 - Synthesis of (3R,2S)-3-propyloxirane-2-carboxylic acid (VIII)

A solution of sodium hypochlorite (10%, 18.5 g, 25 mmol) is added to the solution obtained by mixing crude (3R-*trans*)-(3-propyloxyranyl)methanol (V), obtained as in Example 1 (theoretical yield 116 g, 250 mmol), TEMPO (1.95 g, 12.5 mmol), acetonitrile (350 mL), phosphate buffer (pH = 6.9, 0.7 M, 350 ml) and sodium chlorite (80%, 49.5 g, 550 mmol), and the resulting mixture is stirred vigorously. Aliquots of a sodium hypochlorite solution (5 ml) are added every 60 minutes until complete conversion of the substrate. The end-of-reaction mixture is poured slowly into a mixture of ethyl acetate (300 ml), water (500 ml) and sodium sulphite (60 g) cooled to 0°C, maintaining the pH at around 4 by adding 37% HCl, and the mixture thus obtained is stirred vigorously for 60 minutes. The aqueous phase is then acidified, and the phases are separated. The aqueous phase is extracted with ethyl acetate (100 ml), and the combined organic phases are washed with water (100 mL); the product is then extracted with a solution of NaOH (250 ml) at a pH of around 8, and subsequently with water (100 mL). The basic aqueous phases are combined and acidified, and then extracted with ethyl acetate. The organic phase is treated with a saturated solution of sodium chloride, dried on sodium sulphate, filtered and concentrated at low pressure until (3R,2S)-3-propyloxirane-2-carboxylic acid **(VIII)** is obtained as a colourless oil with a yield of 87% in 2 steps.

¹H-NMR, (CDCl₃) δ: 3.25 (d, 1H, *J* = 1.2 Hz), 3.19 (dt, 1H, *J* = 1.2, 6.9 Hz), 1.71-1.41 (m, 4H), 0.98 (t, 3H, *J* = 7.8 Hz)

### Example 3 - Synthesis of (3S,2S)-3-azido-2-hydroxyhexanoic acid (VII)

NaN3 (50 g, 0.77 mol) and MgSO₄ (89 g, 0.74 mol) are added to the solution obtained by mixing (3R,2S)-3-propyloxirane-2-carboxylic acid **(VIII),** obtained as in Example 2 (92 g, 0.70 mol), methanol (180 mL) and 30% NaOH (80 g, 0.6 mol), and the reaction mixture is maintained under stirring for 18 hours at about 25°C. More NaN₃ (10 g, 0.15 mol) is added, and the reaction mixture is maintained under stirring for a further 8 hours. The end-of-reaction mixture is treated with water (500 mL) and 37% HCl until a solution at pH 5 is obtained; said solution is then extracted with ethyl acetate and the combined organic phases are washed, first with water and finally with a saturated solution of NaCl, and then dried on Na₂SO₄, filtered and concentrated until (3S,2S)-3-azido-2-hexanoic acid **(VII)** is obtained as a yellow oil with a yield of 80%.

¹H-NMR, (CDCl₃) δ: 4.42 (d, 1H, *J* = 3.0Hz); 3.64 (dt, 1H, *J* = 3.0, 9.6Hz); 1.84-1.38 (m, 4H); 0.97 (t, 3H, *J* =7.2 Hz)

### Example 4 - Synthesis of methyl (2S,3S) 3-azido-2-hydroxyethanoate (VII)

A solution of (3S,2S)-3-azido-2-hydroxyhexanoic acid of formula **(VII)** (22 g, 12.7 mmol), prepared as in Example 3 in methanol (100 mL), is treated with a 15% methanolic solution of HCl (11 g, 4.6 mmol). The reaction mixture is maintained under stirring for 18 hours at about 25°C, after which water is added, and the resulting mixture is extracted with ethyl acetate. The organic phase is washed first with a saturated solution of NaHCO₃ and then with a saturated solution of NaCl, dried on sodium sulphate and concentrated to residue. Methyl (3S,2S)-3-azido-2-hydroxyethanoate **(VII)** is obtained as an oil with a yield of 95%; said oil is not purified, but used "as is" in the subsequent reactions.

¹H-NMR, (CDCl₃) δ: 4.33 (d, 1H, *J* = 3.3Hz); 3.84 (s, 3H), 3.54 (dt, 1H, *J* = 3.3, 9.6Hz); 1.75-1.32 (m, 4H); 0.95 (t, 3H, *J*= 7.2 Hz).

### Example 5 - Synthesis of methyl (2S,3S) 3-amino-2-hydroxyethanoate (III)

Methyl (2S,3S) 3-azido-2-hydroxyethanoate **(VII)** (1.2 g, 6.4 mmol), obtained as in Example 4, is dissolved in methanol (12 mL) and treated with 5% Pd/C (0.1 g). The reaction mixture is maintained in hydrogen atmosphere and under stirring at ambient temperature for 6 hours. The end-of-reaction mixture is filtered through a layer of perlite, which is washed with methanol. The solution of methyl (2S,3S) 3-amino-2-hydroxyethanoate **(III)** thus obtained is concentrated at low pressure to a volume of about 6 mL, and used "as is" in the next step. An aliquot is concentrated for analysis purposes.

¹H-NMR, (CDCl₃) δ: 4.14 (d, 2H, *J* = 3.9Hz); 3.77 (s, 3H); 3.08-2.98 (m, 1H); 1.55-1.25 (m, 4 H); 0.91 (t, 3H, *J*= 7.5Hz).

### Example 6 - Synthesis of (2S,3S) 3-amino-2-hydroxy-hexanoyl cylopropylamide (II)

The methyl (2S,3S) **3-amino-2-hydroxyethanoate** solution in MeOH obtained in Example 5 is treated with cyclopropylamine (4.1 g, 72 mmol) and maintained under stirring at 70°C for 18 hours in a hermetically sealed test tube. The end-of-reaction solution is then concentrated at low pressure to give 980 mg of (2S,3S) 3-amino-2-hydroxy-hexanoyl cyclopropylamide **(II)** as a solid, with a yield of 75% in two steps.

### Example 7 - Synthesis of (3R-trans)-(3-propyloxyranyl)methanol (V)

To a stirred solution of Ti(OiPr)₄ (28.22 g, 100 mmol) in dichloromethane (600 mL) cooled to -20°C under inert atmosphere, Na₂SO₄ 33 g, D-(-)-diethyltartrate (24.7 g, 120 mmol) and cumene hydroperoxide (80% w/w, 285 g, 1500 mmol) are added in this order keeping the temperature below -15°C. After 30 minutes, a solution of trans-2-hexen-1-ol (100 g, 1000 mmol) in dichloromethane (200 mL) is added in 20 minutes keeping the temperature below -15°C. After 90 minutes, the reaction mixture is heated to 0°C, water (600 mL) is added, and the resulting mixture is vigorously stirred. After 45 minutes, a solution of NaOH in water (30% w/w, 116 mL) is added and the mixture is vigorously stirred for 30 minutes, then stirring is stopped and the phases are separated. The aqueous phase is extracted with dichloromethane (2 x 100 mL) and the combined organic phases are treated with a saturated solution of sodium thiosulfate (200 mL) for 30 minutes. The phases are separated and the organic phase is treated with a saturated solution of NaCl, the phases are separated, the organic phase is dried on sodium sulfate, filtered and concentrated under reduced pressure to yield crude (3R-trans)-(3-propyloxyranyl)methanol of formula **(V),** which is used in the subsequent reaction as it is.

A part of the crude product was purified by flash chromatography for analytical purposes. The pure sample was analyzed by ¹H-NMR spectroscopy and the obtained data are consistent with the proposed structure. Moreover, the same sample was derivatized as the Mosher ester and the product analyzed by gas chromatography to evaluate the enantiomeric excess (e.e. = 94%).

¹H-NMR, (CDCl₃) d: 3.92 (dd, 1 H, J = 2.7, 12.8 Hz), 3.63 (dd, 1 H, J = 4.5, 12.8 Hz), 2.9-3.0 (m, 2 H), 1.4-1.6 (m, 4 H), 0.94 (t, 3 H, J = 8 Hz).

### Example 8 - Synthesis of (3R,2S)-3-propyloxyranyl-2-carboxylic acid (VIII)

To the solution obtained by mixing crude (3R-trans)-(3-propyloxyranyl)methanol of formula **(V)** obtained as in Example 7 (400 g, 830 mmol as calculated by ¹H-NMR using an internal standard), TEMPO (12.9 g, 83 mmol), acetonitrile (400 mL), phosphate buffer (pH = 6.9, 0.7 M, 1000mL) and sodium chlorite (80%, 187.4 g, 1660 mmol), a solution of sodium hypochlorite in water (9% w/w, 100 g, 124 mmol) is added and the thus obtained mixture is vigorously stirred for about 60 minutes. The mixture is cooled to 0°C and sodium sulfite (230 g) is added. The thus obtained mixture is vigorously stirred for 60 minutes, stirring is stopped and the phases are separated. The aqueous phase is extracted with tert-butylmethylether (200mL) and the combined organic phases are discarded. The aqueous phase is acidified to pH 3 with HCl 37%. The acidic aqueous phase is extracted with tert-butylmethylether (600 mL). The organic phase is treated with a saturated solution of NaCl, the phases are separated and the organic phase is dried on sodium sulfate, filtered and concentrated under reduced pressure to yield (3R,2S)-3-propyloxirane-2-carboxylic acid **(VIII)** as a colorless oil in 70% yield over 2 steps.

¹H-NMR, (CDCl₃) d: 3.25 (d, 1H, J = 1.2 Hz), 3.19 (dt, 1H, J = 1.2, 6.9 Hz), 1.71-1.41 (m, 4H), 0.98 (t, 3H, J = 7.8 Hz)

### Example 9 - Synthesis of (3S,2S)-3-azido-2-hydroxyhexanoic acid (VII)

NaN₃ (29.0 g, 448 mmol) and MgSO₄ (50 g, 424 mol) are added to the solution obtained by mixing (3R,2S)-3-propyloxirane-2-carboxylic acid **(VIII)** obtained as in Example 8 (53 g, 326 mmol calculated by potentiometric titration), acetonitrile (530mL) and NaOH 30% (32.6 g, 244 mmol) and the reaction mixture is heated to 45°C. After 4 hours water (300 mL) and sodium nitrite (13.5 g) are added. The reaction mixture is cooled to 0°C and the pH is adjusted to pH 2 by adding HCl 37%. The phases are separated and the aqueous phase is extracted with ethylacetate (200 mL). The organic phase is firstly washed with water, then washed with a saturated solution of NaCl, the phases are separated and the organic phase is dried on sodium sulfate, filtered and concentrated under reduced pressure to give (3S,2S)-3-azido-2-hexanoic acid **(VII)** as a yellow oil in 89% yield.

¹H-NMR, (CDCl₃) d: 4.42 (d, 1H, J = 3.0Hz); 3.64 (dt, 1H, J = 3.0, 9.6Hz); 1.84-1.38 (m, 4H); 0.97 (t, 3H, J =7.2 Hz)

### Example 10 - Synthesis of methyl (2S,3S) 3-azido-2-hydroxyhexanoate (VII)

A solution of HCl in methanol (30% w/w, 13.5 g, 110 mmol) is added to a stirred solution of (3S,2S)-3-azido-2-hydroxyhexanoic acid of formula **(VII)** (37.5g, 190mmol) obtained as in Example 9 in MeOH (290 mL). After 4 hours the thus obtained mixture is used as it is in the subsequent step. A part of the mixture was concentrated under reduced pressure to give an isolated sample of methyl (3S,2S)-3-azido-2-hydroxyhexanoate which was analyzed by ¹H-NMR.

¹H-NMR, (CDCl₃) d: 4.33 (d, 1H, J = 3.3Hz); 3.84 (s, 3H), 3.54 (dt, 1H, J = 3.3, 9.6Hz); 1.75-1.32 (m, 4H); 0.95 (t, 3H, J = 7.2 Hz).

### Example 11 - Synthesis of methyl (2S,3S) 3-amino-2-hydroxyhexanoate hydrochloride salt (III)

HCl 37% in water (9.8 g) and 5% Pd/C (3 g) are added to a stirred solution of methyl (2S,3S) 3-azido-2-hydroxyhexanoate **(VII)** (34.7 mmol) in methanol obtained as in Example 10. The reaction mixture is kept under an hydrogen atmosphere for 16 hours. The mixture is filtered on perlite and 5% Pd/C (3 g) is added to the filtrate. The obtained mixture is vigorously stirred under an hydrogen atmosphere for additional 8 hours. The end reaction mixture is filtered on perlite, which is washed with methanol. The filtrate is concentrated under reduced pressure to give crude methyl (2S,3S) 3-amino-2-hydroxyhexanoate hydrochloride salt (39 g) as a white solid. The solid is suspended in a mixture of tert-butylmethylether (155mL) and acetone (40mL). The resulting mixture is stirred and heated to 50°C for 2 hours, then cooled to 0°C. The resulting solid is filtered and washed with tert-butylmethylether (80 mL). Methyl (2S,3S) 3-amino-2-hydroxyhexanoate hydrochloride salt **(III)** is obtained as a white solid in 80% yield over 2 steps.

¹H-NMR, (CDCl₃) d: 4.14 (d, 2H, J = 3.9Hz); 3.77 (s, 3H); 3.08-2.98 (m, 1H); 1.55-1.25 (m, 4 H); 0.91 (t, 3H, J = 7.5Hz).

### Example 12 - Synthesis of (2S,3S) 3-amino-2-hydroxy-hexanoyl cylopropylamide hydrochloride salt (II)

Methyl (2S,3S) 3-amino-2-hydroxyhexanoate hydrochloride salt **(III)** obtained as in Example 11 (9.5 g, 48 mmol) is suspended in tetrahydrofuran (50 mL), the mixture is stirred and cyclopropylamine (25g, 440 mmol) is added. After 16 hours the reaction mixture is heated to 40°C and cyclopropylamine is distilled under reduced pressure. Tetrahydrofuran (50 mL) is added and the mixture is cooled to 0°C. After 1 hour the mixture is filtered to obtain 11.5 g of a white solid which is suspended in dimethylformamide (40 ml). The mixture is heated to 70°C until complete dissolution is observed. The solution is distilled under reduced pressure for 3 hours, maintaining the temperature at 70°C. Tetrahydrofuran (75mL) is added to the solution and the mixture is cooled to 20°C. The resulting solid is filtered and washed with tetrahydrofuran, (20mL) to yield 7.5 g of (2S,3S) 3-amino-2-hydroxy-hexanoyl cyclopropylamide hydrochloride salt **(II)** in 70% yield, about 99% HPLC purity and enantiomeric excess > 99% evaluated by HPLC on chiral stationary phase.

## Claims

1. A process for preparing a compound of formula **(II),** either as a single stereoisomer or as a stereoisomer mixture, or a salt thereof, 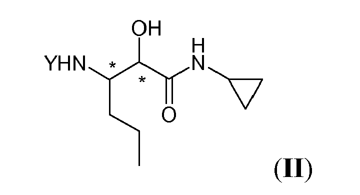
wherein Y is H or an amino protecting group, and the asterisk * indicates the presence of a stereogenic centre with (R) or (S) configuration or a racemic mixture thereof; comprising reacting a compound of formula **(III)** or a salt thereof 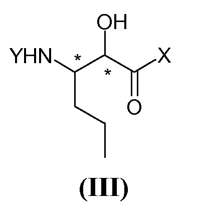
wherein X is an -OR₁ group wherein R₁ is an optionally substituted C₁-C₆ alkyl group, an optionally substituted aryl group or an optionally substituted heteroaryl group; or X is an -SR₂ group wherein R₂ is an optionally substituted C₁-C₆ alkyl group, an optionally substituted aryl group or an optionally substituted heteroaryl group; or X is the reactive residue of a carboxylic acid; and Y and the asterisk * are as defined above; with a cyclopropylamine of formula **(IV),** or a salt thereof, 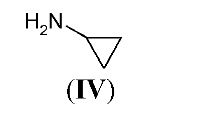
and, if applicable, converting a compound of formula **(II)** to another compound of formula **(II),** and/or to a salt thereof;
said process comprising utilising, as starting material, a compound of formula **(V),** 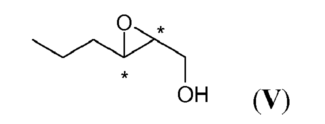
wherein the asterisk * is as defined above, obtained by epoxidation of a compound of formula **(VI),** 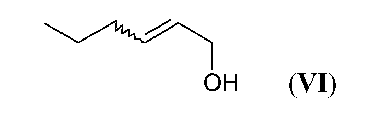
with the double bond in Z or E configuration.

2. A process according to claim 1, wherein the epoxidation reaction is carried out by treatment with an oxidizing agent, preferably a peroxyacid, more preferably m-chloro perbenzoic acid; or an organic peroxide, preferably tert-butyl hydroperoxide, cumyl hydroperoxide or trityl hydroperoxide; or hydrogen peroxide in the presence of acetic acid or a catalyst consisting of a complex of a transition metal, preferably sodium metavanadate or sodium tungstate.

3. A process according to claim 1 or 2, wherein the reaction of a compound of formula **(III)** with a compound of formula **(IV)** is carried out in the presence of a solvent which is a selected in the group comprising a polar aprotic solvent, typically an amide, such as dimethylformamide, dimethylacetamide or N-methylpyrrolidone, preferably dimethylacetamide, acetonitrile or dimethyl sulphoxide; or an acyclic or cyclic ether, such as methyl tert-butyl ether, tetrahydrofuran or dioxane; a chlorinated solvent, such as dichloromethane, dicloroethane, chloroform or chlorobenzene; an apolar aprotic solvent, typically toluene; a polar protic solvent, typically a straight or branched C₁-C₈ alkanol, such as a C₁-C₅ alkanol like methanol, ethanol, isopropanol or tert-butanol; water; a tertiary amine, such as triethylamine and a mixture of two or more, preferably two or three, of said solvents.

4. A process according to claim 3, wherein the reaction of a compound of formula **(III)** with a compound of formula **(IV)** is carried out in the presence of methanol or tetrahydrofuran.

5. A process according to claims 1 to 4, wherein the reaction between a compound of formula **(III)** and a compound of formula **(IV)** is carried out at a temperature between about 0°C and the solvent reflux temperature, preferably between about 40°C and about 80°C.

6. A process according to claim 1, wherein the epoxidation reaction is carried out by enantioselective epoxidation in the presence of an oxidizing agent, and a complex of a titanium salt with an optically active ligand.

7. A process according to claim 6, wherein a titanium salt can be titanium isopropylate, used in stoichiometric or catalytic amounts, preferably in a molar ratio with the substrate ranging between about 20% and about 1%, more preferably ranging between about 10% and 1%.

8. A process according to claim 6, wherein an optically active ligand can be an optically active diol, preferably a tartaric acid derivative, more preferably an ester thereof, in particular methyl tartrate, diethyl tartrate or isopropyl tartrate, used in stoichiometric amounts or catalytic amounts, preferably in a molar ratio with the substrate ranging between about 20% and 1%, more preferably ranging between about 10% and 1%.

9. A process according to claim 1, wherein the epoxidation reaction is carried out at a temperature ranging between about the reflux temperature of the solvent and about -40°C, preferably between about 0°C and about -40°C.

10. A process according to claim 6, wherein the oxidising agent is a peroxide, preferably tert-butyl hydroperoxide, cumyl hydroperoxide or trityl hydroperoxide, used in stoichiometric amounts or in excess, preferably in a molar ratio with the substrate ranging between about 5 and about 1, more preferably between about 2 and about 1.

11. Process according to claims 1-10, wherein the epoxidation reaction is conducted in a solvent inert under the reaction conditions, such as a halogenated solvent like dichloromethane, chloroform or dicloroethane; an apolar aprotic solvent, preferably an aromatic hydrocarbon such as benzene, toluene or xylene, or an aliphatic hydrocarbon such as hexane, heptane or iso-octane; a polar aprotic solvent such as dimethylformamide, dimethylacetamide, acetonitrile or dimethyl sulphoxide; a ketone, such as acetone or methyl isobutyl ketone; an ether, such as tetrahydrofuran or dioxane or a mixture of two or more, preferably two or three, of said solvents.

12. Process according to claim 11, wherein the reaction is conducted in dichloromethane or toluene.

13. A process according to claim 6, wherein a compound of formula (V) is obtained with an enantiomeric excess higher than 90%.

14. A process according to claims 1 and 6, also comprising preparing a compound of formula **(III),** as defined in claim 1, and similarly a compound of formula (**IIIa**) 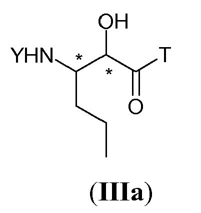
wherein Y and the asterisk * are as defined in claim 1, and T is OH, -OR₁ or -SR₂ wherein R₁ and R₂ are as defined in claim 1, by reducing the azido group in a compound of formula **(VII)** 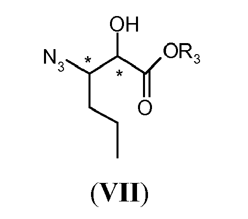
wherein R₃ is H or a C₁-C₆ alkyl group and the asterisk * is as defined in claim 1, and, if applicable, converting a compound of formula (**IIIa**) to another of formula (**IIIa**) or formula **(III);**
wherein a compound of formula **(VII)** is prepared by reacting an epoxy compound of formula **(VIII)** 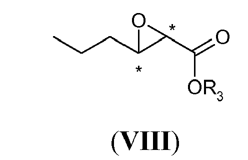
wherein R₃ and the asterisk * are as defined above, with an azide of an alkali metal or alkaline earth metal and, optionally in the presence of a Lewis acid, in a solvent, and, if applicable, converting a compound of formula **(VII)** to another compound of formula **(VII);** and
wherein a compound of formula **(VIII)** wherein R₃ is H is obtained by a process comprising oxidizing a compound of formula **(V)** 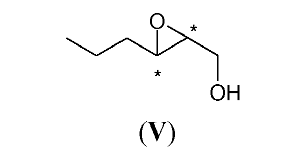
wherein the asterisk * is as defined above.

15. A process for preparing a compound of **(I),** or a pharmaceutically acceptable salt thereof, 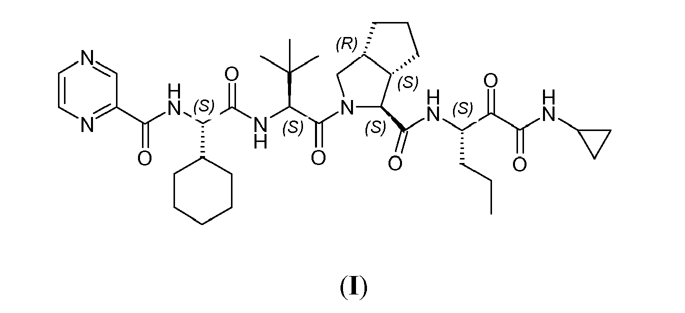 comprising utilising, as starting material, a compound of formula **(V),** 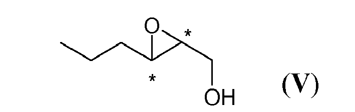 wherein the stereogenic centre indicated by an asterisk * at position 3 has the (R) configuration, obtained by enantioselective epoxidation of a compound of formula (VI), 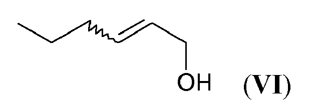 with the double bond in Z or E configuration.
